# EUROPEAN PATENT APPLICATION

(11) **EP 1 947 077 A1**
(43) Date of publication of application: **23.07.2008**
(21) Application number: 06811962.7
(22) Date of filing: 18.10.2006
(51) Int. Cl.: C07C 37/20, C07C 39/16, C07B 61/00

(54) **PROCESS FOR PRODUCING BISPHENOL A WITH SATISFACTORY HUE**

(30) Priority: 21.10.2005 JP 2005307650
(71) Applicant: IDEMITSU KOSAN CO., LTD., Tokyo 100-8321 (JP)
(72) Inventor: YOSHITOMI, Kazuyuki, Ichihara-shi, Chiba 299-0193 (JP); KODAMA, Masahiro, Ichihara-shi, Chiba 299-0193 (JP); MASUDA, Shuichi, Ichihara-shi, Chiba 299-0193 (JP); KOHIRUIMAKI, Jun, Ichihara-shi, Chiba 299-0193 (JP); YAMASAKI, Hokuto, Ichihara-shi, Chiba 299-0193 (JP)
(74) Representative: Vossius & Partner
(86) International application number: PCT/JP2006/320767
(87) International publication number: WO 2007/046434

(57) **Abstract**

An process unit for producing bisphenol A which has (1) a reaction step in which excess phenol is reacted with acetone, (2) a concentration step, (3) a crystallization/solid-liquid separation step, (4) an adduct decomposition step, and (5) a prilling step, wherein those metallic parts of the process unit which come into contact with the reaction mixture in and after the adduct decomposition step (4) are made of a stainless steel containing molybdenum, the temperatures of the raw-material system and the liquid-phase part of the reaction product are regulated to 180°C or lower, and the temperature of a heat carrier for heating these is regulated to 200°C or lower. Due to this, bisphenol A having a outstanding color can be economically and advantageously produced with this process unit for producing bisphenol A from phenol and acetone.

## Description

### Technical Field

The present invention relates to a method of selecting a material for a bisphenol A process unit and a process for producing bisphenol A. More specifically, the present invention relates to a method of selecting a metallic material for a process unit that is used to produce high-quality bisphenol A from phenol and acetone, particularly bisphenol A with outstanding color, and to a process for producing bisphenol A with outstanding color by using the process unit.

### Background Art

Bisphenol A is an important compound as a raw material for epoxy resins or polycarbonates, and the use and demand thereof have increased in recent years. In order to obtain high-quality resins, a high-purity, colorless bisphenol A has been requested.
Bisphenol A is usually produced by reacting phenol and acetone in the presence of an acidic catalyst. The acidic catalyst is exemplified by a strongly acidic cation-exchange resin. The reaction product contains, besides bisphenol A, by-products such as unreacted phenol, unreacted acetone, water generated by the reaction, and colored substances.

As one of the processes for recovering high-purity bisphenol A from the reaction mixture liquid, there is a process of removing the unreacted acetone, the water generated by the reaction, and part of the unreacted phenol from the reaction mixture liquid by distillation and others; cooling the resulting residual concentrated liquid mixture so as to crystallize bisphenol A and phenol as an adduct; removing phenol, after the resulting crystals (adduct crystals) are separated from a mother liquid that contains by-products to recover bisphenol A (for example, see Patent Documents 1 and 2).

In Patent Documents 1 and 2, residual phenol is removed from an adduct of bisphenol A and phenol by steam stripping. The problem is that the color of product bisphenol A becomes degraded because a small amount of a color-causing material is mixed in the product bisphenol A. This problem of color degradation (coloring) was considered inevitable because the color-causing material arises from heating in the process of the adduct crystals and evaporating the resulting melt.
As a process for separating high-purity bisphenol A with outstanding color from the adduct crystals, there is known a process in which the adduct crystals are melted and evaporated after removing oxygen attached to the inside wall of a process unit that includes a melting process unit and an evaporation process unit by cleaning with an organic solvent (for example, see Patent Document 3).

As mentioned above, the production process of bisphenol A may be separated into the following steps: (1) a reaction step in which phenol and acetone are reacted in the presence of an acidic catalyst; (2) a concentration step in which unreacted acetone, water generated by the reaction, and part of unreacted phenol are removed from the resulting reaction mixture liquid by vacuum distillation and others; (3) a crystallization and solid/liquid separation step in which the resulting concentrated reaction mixture liquid is cooled to crystallize bisphenol A in the form of an adduct with phenol, and the resulting crystals (adduct crystals) are separated from a mother liquid that contains by-products; (4) an adduct decomposition step in which the adduct crystals are melted by heating to remove phenol by evaporation and produce bisphenol A; and (5) a prilling step in which bisphenol A thus recovered is produced as prill to obtain product bisphenol A. It is publicly known that stainless steel such as SUS304, SUS304, SUS304L, SUS316, and SUS316L is generally used as a metallic material for the process unit of reaction, concentration, melting, and evaporation. However, there are no clear rules as to which material should be used for which process unit in the above steps so as to produce high-quality bisphenol A. There has been found no document that clearly describes how to determine the operation temperature or heating medium temperature in accordance with the material of a process unit used either.
In an example of Patent Document 3, SUS304 is used as a metallic material for process unites of melting and evaporation. However, washing by injecting high-temperature phenol into the process unites is required to remove oxygen from the inside walls thereof. This operation is extremely troublesome and impractical.

Patent Document 1: Japanese Patent Application Laid-Open No. H02-28126,
Patent Document 2: Japanese Patent Application Laid-Open No. S63-132850,
Patent Document 3: Japanese Patent Application Laid-Open No. H05-39238.

### Disclosure of the Invention

### Problems to be Solved by the Invention

In view of the above circumstances, an object of the present invention is to provide a process for producing economically and advantageously bisphenol A with outstanding color, in a process unit for producing bisphenol A from phenol and acetone, without such a troublesome operation as is described in Patent Document 3.

### Means for Solving the Problems

The present inventors have made intensive studies to achieve the above object, and found that generation of a color-causing material can be prevented by using molybdenum-containing stainless steel (SUS316 or SUS3164L) as a material of the melting and evaporation process units that are used for processing the aforementioned adduct crystals, thereby separating high-purity bisphenol A with outstanding color from the adduct crystals. In addition, it has been found that in a process unit for producing bisphenol A by condensing phenol with acetone, up to the step of crystallization and solid/liquid separation in which the adduct of bisphenol A and phenol is crystallized and separated, general-purpose stainless steel that contains no molybdenum (SUS304 or SUS304L) is used because the color-causing material can be recovered and separated in the mother liquid while the molybdenum-containing stainless steel (SUS316 or SUS316L) that is a high-grade material is used in the adduct decomposition step and subsequent steps thereafter, thereby producing economically and advantageously bisphenol A with outstanding color owing to the reduced plant cost of the process unit for producing bisphenol A. The present invention is accomplished on these findings.

Namely, the present invention provides a process for producing bisphenol A in the following.
1. In a process for producing bisphenol A including: (1) a reaction step in which an excess amount of phenol is reacted with acetone in the presence of an acidic catalyst; (2) a concentration step in which the reaction mixture liquid obtained in the reaction step is concentrated; (3) a crystallization and solid/liquid separation step in which the concentrated liquid obtained in the concentration step is cooled to crystallize an adduct of bisphenol A and phenol, whereby the concentrated liquid is separated into the adduct and a mother liquid; (4) an adduct decomposition step in which phenol is removed from the bisphenol A and phenol adduct so as to recover bisphenol A; and (5) a prilling step in which the recovered bisphenol A is produced as prill so as to obtain product bisphenol A,
   molybdenum-containing stainless steel is used as a metallic material for the equipment that is in contact with the reaction mixture in the adduct decomposition step (4) and subsequent steps thereafter.
2. The process for producing bisphenol A as described in 1, wherein the molybdenum-containing stainless steel is SUS316 or SUS316L.
3. The process for producing bisphenol A as described in 1 or 2, wherein stainless steel that contains no molybdenum is used as a metallic material for the portions that are in contact with the raw materials and reaction products in the steps (1) to (3).
4. The process for producing bisphenol A as described in 3, wherein the stainless steel that contains no molybdenum is SUS304 or SUS304L.

5. A process for producing bisphenol A using the process unit for producing bisphenol A described in 1 or 2, by setting the temperature of the liquid phase of the reaction product at 180°C or lower and the temperature of a heating medium at 200°C or lower in the adduct decomposition step (4) and subsequent thereafter.
6. The process for producing bisphenol A as described in 5, wherein the temperature of the liquid phase of the raw materials and reaction products in steps (1) to (3) is set at 180°C or lower and the temperature of a heating medium for heating the raw materials and reaction products is set at 200°C or lower in the process unit for producing bisphenol A described in 3 or 4.

### Effect of the Invention

In the process for producing bisphenol A according to the present invention, molybdenum-containing stainless steel (SUS316 or SUS316L) is used as a metallic material for the equipment that is in contact with the reaction products in the adduct decomposition step (4) and subsequent steps thereafter, thereby separating high-purity bisphenol A with outstanding color from the adduct crystals and producing easily bisphenol A with outstanding color without troublesome operations such as washing of process unit with high-temperature phenol.

Further, in the steps (1) to (3), general-purpose stainless steel (SUS304 or SUS304L) that contains no molybdenum and is a low cost material is used, thereby reducing the equipment cost. In the adduct decomposition step (4) and subsequent steps thereafter, molybdenum-containing stainless steel (SUS316 or SUS316L) that is a corrosion-resistant high-grade material is used, thereby avoiding degradation of product color, and economically and advantageously producing bisphenol A with outstanding color.

### Best Mode for Carrying Out the Invention

The process for producing bisphenol A according to the present invention includes the steps of: (1) a reaction step; (2) a concentration step; (3) a crystallization and solid/liquid separation step; (4) an adduct decomposition step; and (5) a prilling step in which recovered bisphenol A is produced as prill to product bisphenol A. Thus, bisphenol A with outstanding color is produced.
Hereinafter, each step will be explained in detail.

### (1) Reaction step

The raw material phenol and acetone are reacted in a manner that the amount of phenol is in excess stoichiometrically. The molar ratio of phenol to acetone is preferably phenol/acetone = 3 to 30 and more preferably 5 to 20. The reaction temperature is usually 50 to 100°C. The reaction pressure is usually normal pressure to 1.5 MPa and preferably normal pressure to 0.6 MPa. As a catalyst, a strongly acidic cation-exchange resin such as a sulfonic acid type is usually used.
Further, used in some cases is a catalyst that is given by neutralizing a part of a strongly acidic cation-exchange resin catalyst by an auxiliary catalyst such as a mercapto alkylamine. For example, there may be mentioned a catalyst having sulfonic acid groups neutralized at 5 to 30 mol% by 2-mercaptoethylamine, 3-mercaptopropylamine, N,N-dimethyl-3-mercaptopropylamine, N,N-di-n-butyl-4-mercaptobutylamine, 2,2-dimethylthiazolidine, and the like.
The reaction of phenol with acetone is carried out in a fixed-bed flow process that is a continuous plug-flow process, or in a suspended-bed batch process. In the case of the fixed-bed flow process, the liquid hourly space velocity of the raw material liquid fed to a reactor is about 0.2 to about 50 hr⁻¹. In the case of the suspended-bed batch process, depending on the reaction temperature and pressure, the amount of the resin catalyst is generally in the range of 20 to 100% by mass with respect to the raw material liquid, and the reaction time is about 0.5 to about 5 hrs.

### (2) Concentration step

The reaction mixture obtained in the reaction step is concentrated usually in two steps. In a first concentration step, unreacted acetone, water generated by the reaction, and others are removed by vacuum distillation and the like. The vacuum distillation is carried out usually at a temperature of 30 to 180°C and under a pressure of 13 to 67 kPa. Subsequently, in a second concentration step, phenol is distilled out so as to control the bisphenol A concentration. The bisphenol A concentration is preferably from 20 to 60 % by mass. When the concentration is lower than 20 % by mass, the yield tends to be low. When the concentration is higher than 60 % by mass, the solidification temperature becomes high, thereby bringing about a problem of disabling transportation. Therefore, usually in the first concentration step, the reaction mixture liquid is concentrated in advance so as to control the concentration within the aforementioned range. Usually it is desirable to carry out the second concentration under a pressure of 4 to 40 kPa and at a temperature of 70 to 140°C.

### (3) Crystallization and solid/liquid separation step

The concentrated liquid obtained in the concentration step is cooled from 70 to 140°C to 35 to 60°C so as to crystallize an adduct of bisphenol A and phenol, thereby changing the liquid into slurry. Cooling is carried out with an external heat-exchanger or by heat dissipation through evaporation of water that is added to a crystallization process unit.
Then, the slurry is subjected to solid/liquid separation. The mother liquid obtained in this crystallization and solid/liquid separation step contains the water generated by the reaction, so that the liquid is usually introduced into a dehydration tower. Note that, part of the hydrated mother liquid may be circulated back to the crystallization process unit. After dehydration, the mother liquid usually has such a composition as: phenol in an amount of 65 to 85 % by mass, bisphenol A in an amount of 10 to 20 % by mass, and byproducts such as 2,4'-isomer and the like in an amount of 5 to 15 % by mass. The mother liquid contains a large amount of impurities such as 2,4'-isomer.
The mother liquid may be circulated back to the concentration step after the byproducts contained in the mother liquid are isomerized.
For the isomerization, a sulfonic acid-type cation-exchange resin is usually used. The isomerization is carried out at a reaction temperature of about 50 to about 100°C and, in the case of a fixed bed flow process that is a continuous plug-flow process, at a liquid hourly space velocity of about 0.2 to about 50 hr⁻¹.
Here, in order to avoid accumulation of impurities, it is desirable to subject part of the reaction liquid for the isomerization to blowing and cool the resulting concentrated liquid so as to crystallize and separate the adduct.

The solid obtained after the solid/liquid which contains the adduct as a main component is preferably washed with a cleaning liquid. As the cleaning liquid, there may be used phenol that is recovered by evaporation, raw material phenol, water, a mixed liquid of water and phenol, and the like, and also the same liquid as a phenol solution saturated with bisphenol A.
As the amount of the cleaning liquid used increases, the cleaning efficiency rises naturally, but there is a limit on the amount considering the re-dissolution loss of the crystals, and circulation, recovery and reuse of the cleaning liquid. Usually, cleaning is performed most efficiently when the amount of the cleaning liquid is about 0.1 to about 10 times of the crystals on the mass basis.
After the crystallization and solid/liquid separation, the crystals may be dissolved, and the crystallization and solid/liquid separation may be repeated. The amount of the impurities trapped in the adduct crystals is decreased successively by repeating the crystallization and solid/liquid separation in a multistage.
In this case, as a cleaning liquid for the liquid in the re-dissolution and a cleaning liquid for a solid component that contains as a main component an adduct obtained by solid/liquid separation, there may be used phenol recovered by evaporation, raw material phenol, water, a mixed liquid of water and phenol, and the same liquid as a phenol solution saturated with bisphenol A, in each stage.
There is no particular limitation on the solid/liquid separation process unit that is used for the solid/liquid separation as long as the process unit is conventionally used, and there may be used a belt filter, a drum filter, a tray filter, a centrifugal separator, and the like.

### (4) Adduct decomposition step

The adduct recovered by solid/liquid separation is forwarded to the adduct decomposition step, in which phenol is removed from the adduct to obtain high-purity bisphenol A.
Phenol is removed from the adduct of bisphenol A and phenol that is recovered by solid/liquid separation in the foregoing adduct decomposition step, thereby providing high-purity bisphenol A. Namely, the adduct is heated and melted at about 100 to about 160°C, so that the adduct is decomposed into bisphenol A and phenol. Most of the phenol is removed from the melt with the help of an evaporation drum and the like. Further, residual phenol is removed by steam stripping. In this way, high-quality bisphenol A can be obtained. As to the adduct decomposition temperature, in order to obtain high-quality bisphenol A, the distillation temperature is required to be 180°C or lower and preferably 170°C or lower. In addition, the temperature of a heating medium that is used to the adduct is required to be 200°C or lower and preferably 190°C or lower.

### (5) Prilling step

Bisphenol A obtained in the adduct decomposition step is produced as prill to obtain product bisphenol A, which is stored in a silo and others. The bisphenol A is produced as prill by dropping the melt from a nozzle plate disposed on the upper part of a prilling tower while a cooling gas is allowed to flow from the lower part of the prilling tower toward the upper part, thereby obtaining the prill product (spray granulation).

In the method of selecting materials of the equipment for bisphenol A production according to the present invention, molybdenum-containing stainless steel (SUS316 or SUS316L) that is a high-grade material is used as a metallic material for the portions that are in contact with the reaction products in the adduct decomposition step and subsequent steps thereafter. As shown in the following Examples, in the adduct decomposition step (4), bisphenol A is not colored when the molybdenum-containing stainless steel is used, while bisphenol A is colored when general-purpose stainless steel that contains no molybdenum (SUS304 or SUS304L) is used. It has not been known so far that outstanding color is maintained by using the molybdenum-containing stainless steel in the adduct decomposition step and subsequent steps thereafter.
Note that, the adduct decomposition step and subsequent steps thereafter refer to a metallic material that is used for the portions such as a heat exchanger and a prilling tower nozzle that are in contact with bisphenol A melt, and a mixed liquid of bisphenol A and bisphenol in the adduct decomposition step.
Bisphenol A is well known to be degraded by heat. When bisphenol A is exposed to a high temperature, the quality thereof, particularly color is degraded. Therefore, in the adduct decomposition step and subsequent steps thereafter, the temperature in the liquid phase of the reaction product and the temperature of the heating medium are required to be set at 180°C or lower and 200°C or lower, respectively, and are preferably 170°C or lower and 190°C or lower, respectively.

However, it is not economical to use the high-grade material (for example, SUS316 or SUS316L) for all of the portions as a metallic material in contact with the raw materials and reaction products. In the steps up to the crystallization and solid/liquid separation step (3), the general-purpose stainless steel that contains no molybdenum (SUS304 or SUS304L) can be used. In other words, a heating operation is involved in the vacuum distillation of the concentration step (2) or in the dehydration of the hydrated mother liquid recovered in the crystallization and solid/liquid separation step (3), so that a color-causing material is possibly generated when the general-purpose stainless steel that contains no molybdenum is used. However, the color-causing material is transferred to the mother liquid in the crystallization and solid/liquid separation step (3), so that such impurities as the color-causing material can be removed, thereby avoiding the effect of the color-causing material on bisphenol A even though the color-causing material is generated in the steps up to the crystallization and solid/liquid separation step (3). Therefore, in the steps up to the crystallization and solid/liquid separation step (3), the general-purpose stainless steel that contains no molybdenum (SUS304 or SUS304L) can be used, thereby obtaining excellent bisphenol A with outstanding color in an economical manner.

As mentioned above, the effect of the color-causing material on bisphenol A can be avoided even though the color-causing material is generated in the steps up to the crystallization and solid/liquid separation step (3). However, even in the steps (1) to (3), it is desirable to reduce the amount of the color-causing material generated as much as possible. The temperature of the raw materials and liquid phase of reaction products is desirably set at 180°C or lower and, more desirably 170°C or lower. In addition, the temperature of the heating medium used to heat the raw materials and reaction products is desirably set at 200°C or lower and, more desirably 190°C or lower.
The temperature in the adduct decomposition step (4) and subsequent steps thereafter is as mentioned above. In the adduct decomposition step and subsequent steps thereafter, the high-grade molybdenum-containing stainless steel (SUS316 or SUS316L) is used because it is difficult to purify bisphenol A once the quality of bisphenol A separated by adduct decomposition is degraded. In the adduct decomposition step (4) and subsequent steps thereafter, there is a fewer number of equipment as compared with the steps up to the crystallization and solid/liquid separation step (3), so that the impact on the cost is small even though the high-grade material is used.

### Example

Hereinafter, the present invention will be further described in detail with reference to the following examples, but it should be noted that the present invention is in no way limited by those examples.
In the following Examples, phenol, bisphenol A and others were quantitatively analyzed by a high performance liquid chromatography (HPLC).

### Example 1

### Reaction step:

A cation-exchange resin (Diaion SK104H (trade name), manufactured by Mitsubishi Chemical Corporation) having sulfonic acid groups partly neutralized at 20 mol% by 2-mercaptoethylamine was packed in a fixed-bed reactor tower (material: SUS304), through which phenol and acetone in a molar ratio of 10:1 were passed continuously at an LHSV of 3 hr⁻¹, and the reaction was carried out at 75°C.

### Concentration step:

The resulting reaction mixture liquid was processed under a reduced pressure in a distillation tower (material, SUS304; heating medium temperature, 190°C; liquid temperature, 170°C) to distill out acetone, water, and phenol, unitil the reaction mixture liquid was concentrated to the bisphenol A concentration of 40 % by mass. In this way, a phenol and bisphenol A liquid was obtained.

### Crystallization and solid/liquid separation step:

Water was added to the phenol and bisphenol A liquid having a bisphenol A concentration of 40 % by mass. The liquid was cooled and kept at 50°C under a reduced pressure in a crystallization drum (material: SUS304) so as to crystallize a bisphenol A-phenol adduct, and slurry was obtained.
Then, the resulting slurry was subjected to solid/liquid separation and phenol washing with a solid/liquid separating machine (material: SUS304) to obtain a bisphenol A-phenol adduct. Phenol was added to the bisphenol A and phenol adduct, and the resulting mixture was heated to 170°C (heating medium temperature at 190°C) in a dissolution tank (material: SUS304) to prepare a liquid containing 60 % by mass of phenol and 40 % by mass of bisphenol A.. The liquid was subjected to vacuum cooling crystallization, solid/liquid separation, and phenol washing again with equipment similar as described above, to obtain a bisphenol A-phenol adduct.

### Adduct decomposition step:

The resulting adduct crystals were fed into a melting tank (material: SUS316L) and melted (liquid temperature, 170°C; heating medium temperature, 190°C). Then, the resulting melt was subjected to dephenolization in a distillation tower (material: SUS316L) under a reduced pressure at a liquid temperature of 170°C and a heating medium temperature of 190°C, so that a bisphenol A melt was obtained.

### Prilling step:

The resulting melt was produced prill in an inert gas atmosphere in a prilling tower equipped with a nozzle plate (material: SUS316L) heated with a 190°C heating medium. In this way, bisphenol A prill was produced.
After the resulting bisphenol A was heated at 220°C at air atmosphere for 40 min, the color of the bisphenol A was evaluated by visual inspection using the APHA standard color. The APHA was 10.

### Example 2

The same operations as in Example I were carried out except the heating medium temperature was 210°C and the liquid temperature was 190°C in the distillation of the reaction mixture liquid and the dissolution after crystallization. In this way, bisphenol A prill was produced. The APHA of resulting bisphenol A was 15.

### Example 3

The same operations as in Example 1 were carried out except that the material used for the equipment was changed to SUS316 in the adduct decomposition step and subsequent steps thereafter. In this way, bisphenol A prill was produced. The APHA of the resulting bisphenol A was 10.

### Comparative Example 1

The same operations as in Example 1 were carried out except that the material used for the equipment was changed to SUS304 in the adduct decomposition step and subsequent steps thereafter and that the liquid phase temperature was changed to 190°C and the heating medium temperature was changed to 210°C in the melting, adduct decomposition, and prilling. In this way, bisphenol A prill was produced. The APHA of the resulting bisphenol A was 30.

### Comparative Example 2

The same operations as in Example 1 were carried out except that the material used for the equipment was changed to SUS3 04 in the adduct decomposition step and subsequent steps thereafter. In this way, bisphenol A prill was produced. The APHA of the resulting bisphenol A was 50.

The material used for the equipment in each step, the liquid phase temperature, heating medium temperature, and the evaluation results (APHA) of the color of the product bisphenol A in the above examples and comparative examples are summarized in Table 1. Table 1 shows that bisphenol A with outstanding color is obtained by using SUS316 series stainless steel that contains molybdenum as the material for the equipment in the adduct decomposition step and subsequent steps thereafter, and specifying the heating medium temperature and liquid phase temperature.

[Table 1]

**Table 1**

| | Example 1 | Example 2 | Example 3 | Comparative Example 1 | Comparative Example 2 |
|---|---|---|---|---|---|
| Material for equipment | | | | | |
| Steps (1) to (3) Mo content (mass%) | SUS3040 | SUS3040 | SUS3040 | SUS3040 | SUS304 0 |
| Steps (4) to (5) Mo content (mass%) | SUS316L 2.0 to 3.0 | SUS316L 2.0 to 3.0 | SUS316 2.0 to 3.0 | SUS304L0 | SUS3040 |
| Liquid phase temperature (°C) | | | | | |
| Steps (1) to (3) | 170 | 190 | 170 | 170 | 170 |
| Steps (4) to (5) | 170 | 170 | 170 | 190 | 170 |
| Heating medium temperature (°C) | | | | | |
| Steps (1) to (3) | 190 | 210 | 190 | 190 | 190 |
| Steps (4) to (5) | 190 | 190 | 190 | 210 | 190 |
| Color of product bisphenol A (APHA) | 10 | 15 | 10 | 30 | 50 |

### Industrial Applicability

In the process for producing bisphenol A according to the present invention, bisphenol A with outstanding color can be produced easily without troublesome operations such as washing operation of process unit with high temperature phenol.
Further, in the steps (1) to (3), a general-purpose low-cost material is used, thereby reducing the instrumentation cost. In the adduct decomposition step (4) and subsequent steps thereafter, a high-grade corrosion-resistant material is used, thereby avoiding degradation of product color and economically and advantageously producing bisphenol A with outstanding color.

## Claims

1. A process for producing bisphenol A comprising using, in a bisphenol A process unit including:
(1) a reaction step in which an excess amount of phenol is reacted with acetone in the presence of an acidic catalyst;
(2) a concentration step in which the reaction mixture liquid obtained in the reaction step is concentrated;
(3) a crystallization and solid/liquid separation step in which the concentrated liquid obtained the concentration step is cooled so as to crystallize an adduct of bisphenol A and phenol, whereby the concentrated liquid is separated into the adduct and a mother liquid;
(4) an adduct decomposition step in which phenol is removed from the aduct of bisphenol A and phenol so as to recover bisphenol A; and
(5) a prilling step in which the recovered bisphenol A is produced as prill so as to obtain product bisphenol A,
molybdenum-containing stainless steel as a metallic material for the portions that are in contact with the reaction mixture in the adduct decomposition step (4) and subsequent steps thereafter.

2. The process for producing bisphenol A according to claim 1, wherein the molybdenum-containing stainless steel is SUS316 or SUS316L.

3. The process for producing bisphenol A according to claim 1 or 2, wherein stainless steel that contains no molybdenum is used as a metallic material for the portions in contact with the raw materials and reaction products in the steps (1) to (3).

4. The process for producing bisphenol A according to claim 3, wherein the stainless steel that contains no molybdenum is SUS316 or SUS316L.

5. A process for producing bisphenol A in the bisphenol A process unit according to claim 1 or 2 comprising:
setting the temperature of the liquid phase of the reaction product at 180°C or lower and
setting the temperature of a heating medium at 200°C or lower in the adduct decomposition step (4) and subsequent steps thereafter.

6. The process for producing bisphenol A according to claim 5, wherein the temperature of the liquid phase of the raw materials and reaction products in steps(1) to (3) is set at 180°C or lower and the temperature of a heating medium for heating the raw materials and reaction products is set at 200°C or lower in the bisphenol A process unit according to claim 3 or 4.
